Europäisches Patentamt

European Patent Office

Office européen des brevets

(11) Publication number : **0 440 509 A2**

# EUROPEAN PATENT APPLICATION

(21) Application number : 91300849.6

(22) Date of filing : 01.02.91

(51) Int. Cl.$^5$ : **C12N 5/00**, // C12N5/12

(30) Priority : 02.02.90 GB 9002368
15.06.90 GB 9013374

(43) Date of publication of application :
07.08.91 Bulletin 91/32

(84) Designated Contracting States :
**BE CH DE DK FR GB LI NL**

(71) Applicant : **COMMON SERVICES AGENCY**
**Trinity Park House, South Trinity Road**
**Edinburgh EH5 3SE (GB)**

(72) Inventor : **MacLeod, Alexander James**
**167 Mayfield Road**
**Edinburgh EH9 3AY (GB)**

(74) Representative : **Buchan, Ian Alexander et al**
**Fitzpatricks 4 West Regent Street**
**Glasgow G2 1RS (GB)**

(54) **Novel cell growth medium components and process for producing same.**

(57) The present invention relates to novel cell growth medium components which, when added to cell growth media, can be used as a replacement for part or all of the fetal calf serum which is currently used. The process described in the present application produces cell growth medium components which are both free from active viruses and essentially immunoglobulin-free and are stable on storage. The novel process, which is applied to Cohn fractions of blood or serum, involves suspending the Cohn fraction in chilled physiological saline, adjusting the pH to between 7 and 8, adding polyethylene glycol and then diluting the resultant liquid with water or a suitable buffered saline. Subsequently the resultant solution is pasteurised by heating, this heating being preferably done in the presence of a carbohydrate such as sorbitol. The addition of a reducing agent such as ascorbic acid prior to the pasteurisation improves the stability of the final product.

EP 0 440 509 A2

# NOVEL CELL GROWTH MEDIUM COMPONENTS AND PROCESS FOR PRODUCING SAME

The present invention relates to novel cell growth medium components, and in particular to stable cell growth medium components based on Cohn fraction IV or Cohn fraction II and III combined, of plasma or similar from humans (or other species), and novel processes for producing these novel cell growth medium components. These novel cell growth medium components are particularly suitable for use in media for culture of immortal mammalian cells as a replacement for part or all of the fetal calf serum used currently.

The nutritional requirements of mammalian cells are more stringent than those of most micro-organisms and have not yet been fully defined. Rather than being a free-living organism, a mammalian cell is adapted to a specialised life as part of an organised tissue, dependent on the specialised functions of many other cells and on a circulatory system that ensures a precisely adjusted and stable environment for each cell. Such a cell resists being separated from its tissue and grown in an artificial medium. Most animal cells will not grow at all in suspension ; they grow only when they can attach themselves to a surface. However, techniques have been developed for growing mammalian cells on a small scale in the laboratory.

A mammalian cell culture begins with a mammalian tissue. The tissue is dissociated either mechanically or enzymatically, or by a combination of the two methods, to yield a mixture of single cells and small clumps of cells. The mixture is inoculated into an appropriate liquid growth medium that ordinarily includes salts, glucose, certain amino acids, vitamin and blood serum (usually accounting for between 5 and 20% of the medium).

The serum is included as a means of providing components that have not yet been indentified but have been shown to be needed if the cells are to live and grow in an artificial medium. Fetal calf serum (FCS), which is considered the best available serum for this purpose, is expensive, and its cost largely determines the economic feasibility of a particular culture. Also, there is the problem that the products obtained from cells grown in FCS might be contaminated by bovine proteins, which would be undesirable for human clinical application of the cell products.

Although most mammalian cells need to be anchored to a solid surface, cells that originate in blood or lymphatic tissue, along with most tumour cells and other immortal cells, can be adapted to growth in suspension.

The term "immortal cells" as used in this specification refers to cells which are inherently immortal, for example the tumour-producting myeloma cells, and those which are immortalised, e.g. mammalian cells from various parts of the body which are fused with myeloma cells to give immortal hybridoma cells. Hybridoma cells are, of course, important for their use in producing monoclonal antibodies. Other immortalised cells are those which are transformed by certain viruses, for example, Epstein-Barr virus or are transformed by chemical mutagenenis. Namalva cells, which have been used in the production of interferons, are an example of an inherently immortal cell line arising spontaneously from a tumour found in a girl.

Hitherto immortal cells have had to be grown in a medium comprising a defined component such as RPMI 1640 and an undefined component such as FCS. FCS is, however, expensive.

It would be advantageous to replace serum by some product obtainable from the processing of mammalian blood, particularly human blood. Human blood is processed in large quantities from supplies generated by the blood transfusion service. It is used to prepare fractions of blood plasma, for example, to produce albumins, immunoglobulins and supplies of proteins essential for remedying deficiencies in the blood-clotting chain.

Human blood plasma is conveniently fractionated by the cold ethanol precipitation method. For this method the plasma is treated with ethanol at below 5°C and by increasing the concentration of ethanol and reducing the pH, a succession of fractions are precipitated. The supernatant liquor after each precipitation is subjected to the next. The whole technology is normally described by the umbrella term "Cohn precipitation". It is based on the methods described by E.J. Cohn et al., J. Amer. Chem. Soc., 68, 459-475 (1946), but the original methods therein described have been modified over the years. As an example, see J.G. Watt, Clinics in Haematology, 5, 95-112 (1976) and P. Kistler and H. Friedli in "Methods of Plasma Protein Fractionation", edited by J.M. Curling, Academic Press (1980), pages 3-15. Broadly, the first fraction precipitated contains fibrinogen and fibronectin, the second gamma-globulins, the third beta-globulins, the fourth alpha-globulins and the fifth albumins. The fifth fraction is a large volume product collected at a 40% ethanol concentration and pH 5.2 and represents a commercially valuable product. Each fraction is heavily contaminated by components of other fractions and other materials. Fraction IV can be recovered under various different conditions to give different products. For instance fraction IV can be collected as two cuts, known as IV(1) and IV(4), the latter containing more transferrin and albumins. It should be appreciated that the Cohn fractions referred to herein are obtained from plasma (human or another species) or a similar source.

Many attempts have been made to use components of human blood plasma as a substitute for serum in the culture of mammalian cells. The following account is a selection of the prior art, believed to be representative of various lines of research, in approximate chronological order.

K. Lambert and S.J. Pirt, Develop. biol. Standard, 37, 63-66 (1977) (Joint WHO/IABS Symposium on standardisation of cell substrates for the production of virus vaccines, Geneva, December 1966), reviewed the problems of trying to replace serum as a component of the growth medium for growing human diploid cells. These workers tested the growth factor activity of protein fractions of blood from the Cohn precipitation method. They reported that although growth factor can be recovered, particularly in Cohn fraction IV, the method is poorly reproducible and the fractions could not replace whole serum. They speculated that non-suppressible insulin-like activator (NSILA) or somatomedins contained in blood plasma might have a role as replacements for serum in growing human diploid cells.

J.L. Melnick and C. Wallis, Develop. biol. Standard, 37, 77-82, (1977) pursued the theory that an important function of serum in growth media is its ability to inhibit the proteolytic enzyme trypsin. They tested this theory by trying various media as inhibitors of trypsin. These included albumin human blood plasma Cohn fractions IV, IV(1) and IV(4), fetuin, beta-globulin, gamma-globulin and calf sera. Cells washed thoroughly and cultivated without serum grew to monolayers efficiently only in the presence of serum fraction IV(4) or fetuin.

N.N. Iscover and F. Melchers, Journal of Experimental Medicine, 147, 923-933 (1978) suggested that a mixture of albumin, transferrin and lipids could replace serum entirely for support of lipopolysaccharide (LPS) - stimulated mouse B lymphocytes. However, these results were obtained by the conjoint use of a new medium, a modification of Dulbecco's modified Eagle's medium (DMEM), richer in amino acids and vitamins than the conventional medium RPMI 1640. The cells also required LPS for induction of growth from the resting phase and for their maturation to the stage at which immunoglobulin is secreted.

K. Lambert and S.J. Pirt, J. Cell Science, 35, 381-392 (1979), experimented with an ultrafiltrate fraction of calf serum. This material, molecular weight of 10,000 Daltons or less, supported growth of human diploid cells. It was concluded that a mixture of growth factors such as non-suppressible insulin-like activator (NSILA), somatomedins, epidermal growth factor (EGF) might be responsible.

A thorough study of the use of human blood plasma fractions as growth-stimulating media was made by H. Spieker-Polet et al., Cellular Immunology, 44, 144-156 (1979). These workers found that mouse and rat lymphocytes stimulated by the mitogen concanavalin A showed little growth in a protein-free medium, ample growth when albumin was included in the medium, some growth when human blood plasma Cohn fraction IV was included instead of albumin, but little or no growth from Cohn fraction II, III or IV.

The work of Melnick and Wallis, supra, was pursued by A.J. MacLeod and O. Drummond, Develop. biol. Standard, 46, 17-20 (1980). These authors analysed various sera and some protein solutions prepared from the by-products of human blood plasma fractionation. A fraction IV (4) solution and a solution prepared from plasma depleted in coagulation factor, but otherwise whole, were reported to support cell growth at high cell densities. The kind of cells is not stated but were in fact skin fibroblasts.

An article by A.J. MacLeod, Nature, 285, 136-137 (1980) discussed ways of producing human proteins including the use of recombinant DNA technology and transformation of mammalian cells including preparation of hybridoma cell lines. The author suggests that by-products of the fractionation of human plasma could provide material for supporting the growth of human cells cultured in vitro. No more specific suggestion is given, but the author comments that the by-products consist largely of alpha- and beta-globulins and low molecular weight proteins and these have already been shown to contain many of the specific components required for the culture.

R. Weinstein et al., Journal of Cellular Physiology, 110, 23-28 (1982) describe a serum-free medium for cultivating normal human foreskin fibroblasts (NHFF) in vitro. These workers experimented with a medium containing acid- and heat-extracted Cohn fraction IV. The acid- and heat-extraction steps are for the purposes of producing somatomedins. (The paper refers to this material simply as "CF IV", but it is clear that this heat- and acid-extracted material, rather than the complete fraction, is intended). The authors found that NHFF growth did not decrease when the "CF IV" somatomedins were omitted. In the end, they recommended a medium which they designated "Medium F", lacking "CF IV" and consisting of insulin, transferrin, thrombin, hydrocortisone, ovalbumin, ascorbic acid, trace elements and DMEM.

T. Kawamoto et al., Analytical Biochemistry, 130, 445-453 (1983), describe a serum-free medium for cultivation of mouse myeloma cells which they designate "KSML" and which consists of a 2 :1 :1 mixture (by volume) of RPMI 1640, DMEM, another defined medium known as Ham's F12, with L-glutamine, sodium pyruvate, glucose, penicillin, ampicillin and streptomycin, supplemented with crystalline bovine insulin, transferrin (free of $Fe^{3+}$ ions), 2-aminoethanol, 2-mercaptoethanol, sodium selenite, human low-density lipoprotein and oleic acid in a complex with crystalline fatty acid-free BSA. This work follows in the line of research of Iscove and Melchers, supra.

C.A. Conover et al., Journal of Cellular Physiology, 116, 191-197 (1983) studied growth and replication of human fibroblasts in the presence of human hypopituitary serum (HHS). Experiments were conducted with a medium containing a sub-fraction of Cohn fraction IV(1), this sub-fraction containing insulin-like growth factor-1, otherwise known as somatomedin C (SM-C). Cells were grown in DMEM containing 20% fetal calf serum (FCS), the FCS being replaced by various amounts of SM-C. The SM-C was not by itself (in the absence of HHS) a substitute for the FCS. In the presence of 1% HHS, the SM-C did sustain cell growth, although not as well as FCS.

It will be apparent that there is a great deal of contradiction and uncertainty in the prior art in relation to the role of blood plasma constituents. For example, some researchers suggest that Cohn fraction IV(4) promotes cell growth, e.g. MacLeod and Drummond (1980) and Melnick and Wallis (1977), other authors have concentrated attention on other sub-components of fraction IV such as somatomedins, e.g. Lambert and Pirt (1977 and 1979), and Conover (1983), while Spieker-Polet (1979) et al., reject fraction IV as yielding a useful growth factor and Weinstein (1982) et al. reject fraction IV as a component of their optimum serum-free product.

UK Patent Application No. 2,150,595, disclosed a process for producing a cell growth medium component and the novel cell growth medium component itself based on either Cohn fraction IV or Cohn fraction II and III. The novel cell growth medium components claimed in this patent application were preferably devoid of gamma-globulin, normally present in Cohn fraction IV and Cohn fraction II and III, and were produced in a process where the temperature was carefully kept at a low level, typically less than 10°C. Unfortunately the cell growth medium component produced by the processes claimed tended to be unstable on storage, thus being of limited value for commercial purposes and were also not guaranteed to be free from viruses and in particular active viruses. This latter problem, particularly in view of present concerns about Hepatitis virus and Human Immunodeficiency Virus (HIV), which could conceivably be present in the blood of the human donors from which the plasma is obtained, which is then used to prepare the cell growth medium components, meant that these cell growth medium components could not be used in any cell culture system which in turn was used to produce products, e.g. monoclonal antibodies, which could be used in humans since the risk of infection by these viruses in the cell growth medium component could not be excluded. Furthermore the previously known cell growth medium components produced from Cohn fraction IV were contaminated by immunoglobulins which, if the cell growth medium component is used to support the growth of cells for the production of monoclonal antibodies, can contaminate the monoclonal antibodies so produced.

Accordingly it is an object of the present invention to obviate or mitigate these problems by providing a novel, stable, active virus free cell growth medium component and a novel process for preparing same.

According to the present invention there is provided a novel cell growth medium component produced from a Cohn fraction IV or a Cohn fraction II and III combined, characterised in that the said novel cell growth medium component is substantially free of immunoglobulins.

According to a further aspect of the present invention there is provided a novel cell growth medium component that is further characterised in that it is stable on storage and contains no active viruses.

According to a yet further aspect of the present invention there is provided a process of producing a substantially immunoglobulin free novel cell growth medium component from a Cohn fraction IV or Cohn fraction II and III combined characterised in that the process contains the following sequential steps.

a) resuspension of the frozen Cohn fraction in about twice its own weight of chilled physiological saline 0.9% (W/V) Nacl or some other suitable diluent.

b) adjusting the pH of the resultant liquid to between about pH 6.0 to pH 12.0.

c) adding polyethylene glycol (PEG) 5-15% (w/v), or another known method of precipitating proteins and in particular immunoglobulins.

d) diluting the resultant liquid with about two to three volumes of preferably either pyrogen free water or phosphate buffered saline.

According to a yet further aspect of the present invention there is provided a process of producing a stable and active virus free novel cell growth medium component from a Cohn fraction IV or a Cohn fraction II and III combined characterised in that the novel cell growth medium component resulting from step d) above is subject to the following step

f) heating the resultant solutions under conditions equivalent in terms of heat input or of viral kill achieved to at least plus 60°C for 10hrs, the resultant solution containing a novel cell growth medium component may be cloudy when made up in phosphate buffered saline (step d) and was found to be stable when diluting the cell structure medium for at least one week at room temperature. In a further preferred embodiment a stabiliser including for example carbohydrates, amino acids or other substances known to those skilled in the art, e.g.sorbitol at 90 grams per litre, (step e) could be added prior to (step f). This gave a novel cell growth medium component after (step f) that was stable for periods of at least six months at 4°C. This novel cell growth medium component, when diluted in a cell culture medium is stable for about one week even at temperatures as high as +20°C. Sorbitol concentrations higher than 90 g/l could be employed.

In yet a further aspect of the present invention suitable reducing agents including, for example, L-absorbic acid or β mercaptoethanol can be added to the solution after stage d) above at concentrations between about 0.6mM and 40mM to improve the stability of the product of steps e) and f).

In yet a further aspect of the present invention there is provided a novel process for cultivating cells and producing products from cells cultivated in novel cell growth medium components of the present invention.

At step (e) above suitable carbohydrates other than sorbitol can be used. These other carbohydrates include for example sugars such as sucrose and manitol. The concentrations of these would need to be adjusted appropriately to obtain the desired effects.

It should be appreciated that when Cohn fraction IV or Cohn fractions II and III are mentioned throughout this patent application it is to be taken as including cold ethanol fractions produced by the Cohn fraction process or the various well known variants of Cohn fractions produced from blood or plasma by the various variants of this Cohn process including greater fractions containing these fractions e.g. II, III and IV or IV and V. It should be noted that this present novel process is most suitable for the purification of novel cell growth medium components from Cohn fractions IV (I) or Cohn fractions IV (4) or equivalents thereof.

The term substantially free from immunoglobulins or substantially immunoglobulin free is to be taken herein to mean that the preparation which results from step d of a novel process described herein has an immunoglobulin content of preferably less than 2 grams per litre, more preferably less than 1 gram per litre and most suitable has about 0.1 gram per litre immunoglobulin or less.

Preferably the polyethylene glycol used has an average molecular weight of about 4,000 MW and is used as about a 50% (W/V) solution although it will be appreciated that other polyethylene glycols including, for example, PEG 6,000 can be used in which case their concentration would have to be adjusted accordingly. Preferably at step (b) above the pH will be adjusted to between about pH6.0 and pH12.0, more preferably to between about pH 7.2 and pH7.6 and most suitably to about pH 7.4.

Preferably at step (c) above the PEG will be in a final concentration of between 9 and 11%(w/v) and most suitably will be in a final concentration of 10% (w/v) when using a 50% (w/v) solution of PEG-4000 in water although it will be appreciated that other final concentrations can be used particularly if PEG's of different molecular weight are used.

It will be appreciated, by a person skilled in the art, that suitable well known alternative diluents can be employed instead of physiological saline 0.9% (w/v) at step (a) and instead of pyrogen free water or phosphate buffered saline at step (d) above. For example other buffers including, for example, those such as Tris or citrate buffers can be employed at step (d) above.

Compared with known processes for preparing novel cell growth medium components the present process provides novel cell growth medium components from Cohn Fractions of blood which are stable, virus free, and have a lower final level of PEG and immunoglobulins. It is thought that this lower final level of PEG, previously known processes having about 10% (v/w) final concentration before they are used as a cell growth medium component, compared with the present concentration of about 5% (v/w) final concentration with the present novel process and the present novel cell growth medium components before they are used as a cell growth medium component aids in the stability of the products of the present process particularly when subjected to pasteurisation to destroy viruses

One novel process of the present invention will now be described by way of example only and without limitation to the present invention.

Example 1 - Novel Process

A Cohn fraction IV-1 was used in this novel process. Briefly this is the fraction of the plasma collected at pH 5.2 and with 21% (v/v) ethanol. The paste of precipitated proteins and occluded liquor could either be used immediately or after storage at below -20°C, it having been found that storage of the paste at temperatures between 0oC and -20oC is not particularly acceptable ; however it was found that pastes which had been frozen and thawed were more easily resuspended for processing.

Total protein content was monitored by measuring $E_{280}$ and specific proteins measured by immuno-nephelometry. The protein profile of preparations was obtained by HPLC gel filtration through a TSK G3000W column and by electrophoresis through polyacrylamide gels.

The resultant paste was treated as follows.

Step A

The process developed for immunoglobulin depletion of Fraction IV-1 protein solution was to resuspend the frozen and thawed Fraction IV-1 paste in twice its own weight of chilled physiological saline, 0.9% (w/v) NaCl. If pure water was used for this the subsequent addition of PEG-4000 failed to produce a precipitate that could be collected by centrifugation.

Step B

The pH of the Fraction IV-1 suspension was adjusted to 7.4 ± 0.2 by the addition of about 3.8g NaOH per Kg of the original paste.

## Step C

50% (w/v) PEG-4000 was added to this to give 10% (w/v) PEG-4000 final concentration. The precipitate was removed by centrifugation at about 4500G for 20 minutes at + 20°C. The PEG is used to deplete the levels of immunoglobulin.

## Step D

The PEG-supernatant immunoglobulin depleted fraction IV-1 protein solution could be diluted with two volumes of either pyrogen free water or phosphate buffered saline. This was found to reduce the PEG concentration sufficiently to prevent precipitate formation on freezing and thawing and to give stability during storage at -20°C. This novel cell growth medium component product contained about 7g total protein/litre, this protein including about 5 grams of albumin , 0.5 grams of transferrin and 0.1 grams of immunoglobulin per litre with other unidentified proteins. The immunoglobulin was reduced from about 3 grams per litre in the re-dissolved total fraction IV-1 protein. The final preparation would support suspension culture of hybridoma cells adapted to these conditions when 2.5% (v/v) was used to supplement RPMI 1640 medium.

## Pasteurisation

Heating stabilised human albumin in aqueous solution at +60°C for 10h has been shown to be an exceptionally effective method for inactivating viruses that may contaminate it.

The product from step d can be then sterilised by filtration through a 0.2 μm filter. Unlike normal serum this product does not require several polishing filtration steps prior to filtration through a 0.2 μm filter.

When immunoglobulin-depleted fraction IV-1 novel cell growth medium solution that had been finally diluted with water and without additional stabiliser was heated at +60°C for 10h the product remained clear and cells would still grow in medium supplemented with it. However it was found that medium containing this pasteurised product had to be prepared immediately before use as there was a substantial loss of cell growth promoting activity if the complete medium was stored for as little as 24 hours at +37°C. If the final dilution was made with phosphate buffered saline the medium containing the product would be stable for at least a week at + 20°C but if the product was cloudy it can make downstream processing of protein produced in the cell culture more difficult.

Experiments have been carried out to compare the effects of the pasteurisation process on viral inactivation both in novel cell growth medium components of the present invention and in human albumin fractions which have conventionally been used as cell growth components in cell tissue media. These experiments were carried out by incorporating various viruses such as herpes simplex, mumps virus, semliki forest virus, vaccinia and phages T4 and MS-2. In the tests the novel cell growth medium components of the present invention and the human albumin fractions previously known were inoculated with these viruses and phages and then subjected to the pasteurisation process described above. Sequential samples were then taken from the various test materials to determine the number of viable viruses and phages left after various periods of heating at 60°C. In all cases it has been shown that the viruses were very rapidly inactivated, in both the novel cell growth medium components of the present invention and in the human albumin fractions previously known, and in some cases the viruses have been inactivated by the time the solutions have reached 60oC. It is therefore clear, from these results, that the novel cell growth medium components of the present invention, once they have been treated by the pasteurisation process above, are free from any active viruses or phages and thus are safe to use in cell growth media which may subsequently be used to produce products for the treatment of humans.

## Step E, Step F

Surprisingly it was found that formation of a haze could be prevented by the addition of about 90g of sorbitol/1 to the novel cell growth medium solution after it had been diluted with either water or phosphate buffered saline. The resulting pasteurised product was sufficiently stable after dilution in the defined medium component in which cells are grown to allow preparation of the complete medium including the novel cell growth medium component of the present invention, for at least a week before use.

There was a distinct change in the colour of the preparation from yellow/green to blue/green associated with loss of adsorbance at 450nm when the immunoglobulin-depleted fraction IV-1 protein solution was pasteurised. To try to prevent this change occurring samples were heated supplemented with L-ascorbic acid or with β-mercaptoethanol. Concentrations of each in doubling dilutions from 40mM to 0.31mM were evaluated. Neither 0.31 nor 0.62mM of either reducing agent prevented the change in colour to blue/green on heating but at higher concentrations the product was yellow after heating although the pattern of adsorbance at wave-lengths between 320nm and 630nm was not the same as that of unheated material.

The heated product was completely clear at all concentrations of ascorbate used and supported normal cell growth. β-mercaptoethanol produced opalescence at 2.5 and 5.0mM and at 10mM and higher the product was turbid and the cells failed to grow at all in medium containing these preparations.

## USE OF NOVEL CELL GROWTH MEDIUM COMPONENT IN A CELL CULTURE SYSTEM

The cell line used to test novel cell growth medium components in this study was RFHBs1 murine-murine hybridoma producing monoclonal antibody against Hepatitis B surface antigen. Initial cell growth tests were made in 25cm² tissue culture flask containing 10ml of medium and suspension culture tests in Techne 125ml MCS cell culture vessels. Viable cell counts were made by haemocytometer after straining the cells with MTT. Antibody levels were measured by ELISA against a mouse serum standard.

Every cell line with which it has been attempted has grown well in medium supplemented with the novel cell growth medium components of the present invention, although the process of adaption has itself sometimes been protracted and has to be considered a major step on the way to routine use of this type of product. To adapt them subcultured cells growing in 5% (v/v) calf serum in tissue culture flasks were placed into the same basal medium supplemented with 5% (v/v) calf serum and 10% (v/v) solution of a novel cell growth medium component of the present invention at a cell density preferably not less than 1 x 10⁵ cells/ml. The cells were passaged frequently so that they did not enter the stationary phase and over a period of up to 8 to 10 weeks the amount of calf serum in the medium was reduced in steps of 1% (v/v) until the cells were growing in medium supplemented with a novel cell growth medium component of the present invention alone.

Cell banks were established at this stage by freezing the cells in a mixture of 10% (v/v) dimethylsulphoxide and 90% (v/v) stable plasma protein solution, a preparation of about 88% pure human albumin at 45g total protein/1.

The cells grew readily in medium with a novel cell growth medium component in stirred vessels but again careful preparation of the culture was required. The cells were thawed and put back into culture in the first place in a tissue culture flask. When the viable cell density had at least doubled the cells were transferred to a stirred vessel, the cell density being kept preferably above 1 x 10⁵ cells/ml after this first inoculation. Once adapted to growth in stirred reactors the cells could be harvested and frozen in liquid nitrogen and could usually be restored directly to a stirred vessel on thawing.

It has been observed that in medium supplemented with pasteurised novel cell growth medium component that some of the cells may gather into aggregates of 3 or 4 or of many thousands of cells. This happens in both static and stirred cultures. The cells in the aggregates were viable at first but if very large aggregates formed those in the centre died. The aggregates could usually be disrupted easily by gentle pumping in and out of a pipette. Once disrupted the aggregates did not reform to the same extent and as the cells adapted completely to growth in medium supplemented with pasteurised novel cell growth medium component solution the tendency to aggregate disappeared.

Once established in stirred culture RFHBs1 cells could be maintained in growth by a 10% (v/v) solution of a novel cell growth medium component and at this level the medium would have contained about 500mg albumin, 25mg transferrin and 3mg immunoglobulin per litre.

Data from preliminary studies suggests that a novel cell growth medium component derived from Cohn fraction IV - 4 is equally effective in supporting cell growth and given its much more equal content of albumin and transferrin it may make it possible to achieve cell growth with a very much lower total protein content in the culture medium.

## Claims

1. A novel cell growth medium component produced from a Cohn fraction IV or Cohn fraction II and III combined, characterised in that the said novel cell growth medium component is substantially free from immunoglobulins.

2. A novel cell growth medium component as claimed in Claim 1, characterised in that it is stable on storage and contains no active viruses.

3. A process for producing a novel cell growth medium component as claimed in Claim 1, characterised in that the process contains the following sequential steps :
   a) resuspension of the frozen Cohn fraction in about twice its own weight of chilled physiological saline 0.9% (W/V) Nacl or some other suitable diluent.
   b) adjusting the pH of the resultant liquid to between about pH 6.0 to pH 12.0.
   c) adding polethylene glycol (PEG) to 5-15% (w/v), or another known method of precipitating proteins and in particular immunoglobulins.
   d) diluting the resultant liquid with about two volumes of preferably either pyrogen free water or phosphate buffered saline.

4. A process for producing a novel cell growth medium component as claimed in claim 3 wherein the pH of the resultant liquid is adjusted to between about pH 7.2 and 7.6.

5. A process for producing a novel cell growth medium component as claimed in claim 3 characterised in that polythylene glycol is added to about 9-11% weight volume.

6. A process for producing a novel cell growth medium component as claimed in claim 2 and using the process claimed in Claim 3, characterised in that the solution from step d is subjected to the following process :
   f) heating the resultant solution to the equivalent of at least + 60°C for 10hrs.

7. A process for producing a novel cell growth medium component as claimed in Claim 3, characterised in that a carbohydrate (step e) is added prior to the heating step (f) as claimed in Claim 6.

8. A process for producing a novel cell growth medium component as claimed in Claim 3, characterised in that a reducing agent can be added after stage d.

9. A process for producing a novel cell growth medium component as claimed in Claim 7, characterised in that the carbohydrate is a sugar.

10. A process for producing a novel cell growth medium component as claimed in Claim 9, characterised in that the carbohydrate is sorbitol and it is added at about 90 g/l.

11. A process for producing a novel cell growth medium component as claimed in Claim 8, characterised in that the reducing agent is L-absorbic acid or β mercaptoethanol at concentrations of about 0.6mM to 40mM.

12. A process for producing a novel cell growth medium component as claimed in Claim 3, characterised in that the polyethylene glycol used has an average molecular weight of about 4,000.

13. A process for producing a novel cell growth medium component as claimed in Claim 3, characterised in that the pH in step b is between pH7.2 and pH7.6 and most suitably about pH 7.4.

14. A novel process for cultivating cells producing products from cells, characterised in that the cell culture medium contains novel cell growth medium components as claimed in Claim 1 or 2 or produced according to any of the Claims 3 to 13 above.